# EUROPEAN PATENT APPLICATION

(11) **EP 3 871 596 A1**
(43) Date of publication of application: **01.09.2021**
(21) Application number: 19876432.6
(22) Date of filing: 03.10.2019
(51) Int. Cl.: A61B 5/0408

(54) **BIOELECTRODE, BIOLOGICAL SENSOR, AND BIOLOGICAL SIGNAL MEASUREMENT SYSTEM**

(30) Priority: 26.10.2018 JP 2018202286
(71) Applicant: Sumitomo Bakelite Co.Ltd., Shinagawa-ku Tokyo 140-0002 (JP)
(72) Inventor: KITAZOE Katsuma, Tokyo 140-0002 (JP); YAGISAWA Takashi, Tokyo 140-0002 (JP); UENO Masaomi, Tokyo 140-0002 (JP); HARADA Takuya, Tokyo 140-0002 (JP)
(74) Representative: Ter Meer Steinmeister & Partner
(86) International application number: PCT/JP2019/039170
(87) International publication number: WO 2020/085034

(57) **Abstract**

A biomedical electrode according to the present invention includes: a plate-shaped support portion; a plurality of elastic pillar portions that are provided on a first surface of the plate-shaped support portion; and a conductive resin layer that is formed to cover distal ends of the elastic pillar portions, in which the plurality of elastic pillar portions are arranged to surround a center portion of the first surface of the plate-shaped support portion, each of distal end portions of the plurality of elastic pillar portions has an inclined surface that is inclined with respect to the first surface, and when a point of the inclined surface closest to the first surface is represented by P1, a point of the inclined surface farthest from the first surface is represented by P2, and a half line extending from P2 to P1 is represented by L, the point P1 is present at a position closer to the center portion than the point P2 in the plurality of elastic pillar portions, and the half line L is configured to pass through the center portion or a vicinity of the center portion in the plurality of elastic pillar portions.

## Description

### TECHNICAL FIELD

The present invention relates to a biomedical electrode, biomedical sensor, and a biomedical signal measurement system.

### BACKGROUND ART

Various biomedical electrodes have been developed until now. As such a technique, for example, a technique described in Patent Document 1 is known. Patent Document 1 describes an electroencephalographic electrode including: a base material that is formed of an elastic body; and a protrusion portion that is formed on a surface of the base material (claim 1 in Patent Document 1). Patent Document 1 describes that, since the protrusion portion has a circular or polygonal pillar shape, a distal end in contact with a scalp is flat (claim 1, paragraph 0046, and Fig. 1 of Patent Document 1).

### RELATED DOCUMENT

### PATENT DOCUMENT

[Patent Document 1] Japanese Laid-open patent publication NO.

### SUMMARY OF THE INVENTION

However, as a result of a thorough investigation, the present inventors found that there is a room for improvement of the biomedical electrode described in Patent Document 1 from the viewpoint of measurement stability.

It was found that, in a case where a biomedical electrode formed of a silicone rubber of which a distal end includes a plurality of flat pillar portions is pressed against a curved surface or a flat surface, there is a variation in a deformation direction of the pillar portion, and the contact area with a measurement target varies depending on uses. When there is a variation in the contact state or contact area with the pillar portion during contact with a measurement target, there may be a concern that a measured value cannot be stably obtained.

Accordingly, the present inventors further conducted an investigation and found that, by cutting distal end portions of a plurality of pillar portions in a biomedical electrode such that the height increases from the inside toward the outside, the plurality of pillar portions are deformed to spread from the inside toward the outside during contact with a measurement target, and a variation in the contact states of the pillar portions can be suppressed.

The present inventors further conducted a thorough investigation based on the findings and found that, by arranging the plurality of pillar portions to surround a center portion of a first surface of a plate-shaped support portion and forming an inclined surface in the distal end portion of the pillar portion such that the height on the outside is higher than on the inside, a variation in the deformation direction of the plurality of pillar portions can be suppressed, and the measurement stability of the silicone rubber biomedical electrode can be improved, thereby completing the present invention.

According to the present invention, there is provided a biomedical electrode including:
a plate-shaped support portion;
a plurality of elastic pillar portions that are provided on a first surface of the plate-shaped support portion; and
a conductive resin layer that is formed to cover distal ends of the elastic pillar portions,
in which the plurality of elastic pillar portions are arranged to surround a center portion of the first surface of the plate-shaped support portion,
each of distal end portions of the plurality of elastic pillar portions has an inclined surface that is inclined with respect to the first surface, and
when a point of the inclined surface closest to the first surface is represented by P1, a point of the inclined surface farthest from the first surface is represented by P2, and a half line extending from P2 to P1 is represented by L,
the point P1 is present at a position closer to the center portion than the point P2 in the plurality of elastic pillar portions, and
the half line L is configured to pass through the center portion or a vicinity of the center portion in the plurality of elastic pillar portions.

In addition, according to the present invention, there is provided a biomedical sensor including the above-described biomedical electrode.

In addition, according to the present invention, there is provided a biomedical signal measurement system including the above-described biomedical sensor.

The present invention provides a biomedical electrode having excellent measurement stability, and a biomedical sensor and a biomedical signal measurement system including the biomedical electrode.

### BRIEF DESCRIPTION OF THE DRAWINGS

The above-described object and other objects, characteristics, and advantageous effects will be further clarified using the following preferred embodiment and the following accompanying drawings.

Figs. 1A and 1B are schematic diagrams showing the summary of a biomedical electrode according to an embodiment, in which Fig. 1A is a perspective view and Fig. 1B is a cross-sectional view taken along line A-A of Fig. 1A.
Fig. 2 is a schematic diagram showing a biomedical sensor according to the embodiment.

### DESCRIPTION OF EMBODIMENTS

Hereinafter, an embodiment of the present invention will be described using the drawings.

An embodiment will be described by defining front, rear, right, left, upper, and lower directions as shown in the drawings. However, the directions are defined for convenience of easy understanding of a relationship between components. Accordingly, this definition does not limit directions during the manufacturing or use of a product according to the present invention.

In all the drawings, the same components will be represented by the same reference numerals, and the description thereof will not be repeated. In addition, the drawings are schematic diagrams, in which a dimensional ratio does not match the actual one.

In this specification, the term "substantially" includes a range in consideration of a tolerance, a variation, or the like during manufacturing unless explicitly specified otherwise. A range represented by "to" includes an upper limit value and a lower limit value unless explicitly specified otherwise.

A biomedical electrode according to an embodiment will be briefly described.

The biomedical electrode includes: a plate-shaped support portion; a plurality of elastic pillar portions that are provided on a first surface of the plate-shaped support portion; and a conductive resin layer that is formed to cover distal ends of the elastic pillar portions. In the biomedical electrode, the distal end portion of each of the plurality of elastic pillar portions has an inclined surface that is inclined with respect to the first surface, and when a point of the inclined surface closest to the first surface is represented by P1, a point of the inclined surface farthest from the first surface is represented by P2, and a half line extending from P2 to P1 is represented by L, the point P1 is present at a position closer to the center portion than the point P2 in the plurality of elastic pillar portions, and the half line L is configured to pass through the center portion or a vicinity of the center portion in the plurality of elastic pillar portions.

The present inventors obtain the following findings.

A direction in which the pillar portion of the biomedical electrode is pressed against a measurement target will be referred to as "pressing direction". It was found that, in a case where a distal end of the pillar portion configures a flat surface in the pressing direction, when coming into contact with a measurement target, the plurality of pillar portions are deformed in various directions, and there is a variation in the deformation direction. The same can also be applied to a case where a measurement surface of a measurement target is a curved surface. It is presumed that a variation in the shape of the measurement surface of the measurement target, the operation of an operator, or the like causes the deformation in various directions.

On the other hand, it was found that, in a case where distal end portions of a plurality of elastic pillar portions configure an inclined surface with respect to the pressing direction, when coming into contact with a measurement target, the plurality of elastic pillar portions are deformed to spread from the inside toward the outside, and a variation in the deformation direction can be suppressed. In addition, even in a case where a variation in the shape of the measurement surface or the operation of an operator occurs to some extent, a variation in the deformation direction of the plurality of pillar portions can be suppressed.

The present inventors further conducted a thorough investigation based on the findings, by forming the inclined surface that is configured to increase from the inside toward the outside of a plurality of elastic pillar portions in the distal end portions of the plurality of elastic pillar portions that are arranged to surround a center portion of a first surface of a plate-shaped support portion, a variation in the deformation direction of the plurality of pillar portions can be suppressed such that the measurement stability in the biomedical electrode can be improved.

The biomedical electrode according to the embodiment can detect a variation in bioelectrical potential such as brain wave, heart beat, muscular activity, or nervous system activity. The biomedical electrode can further include a connector or an electronic component to configure a biomedical sensor that can be connected to an external apparatus. This biomedical sensor is wearable. By analyzing the bioelectrical potential such as a brain wave detected from the biomedical sensor, a biomedical signal measurement system corresponding to various uses can be constructed.

Hereinafter, the biomedical electrode according to the embodiment will be described below.

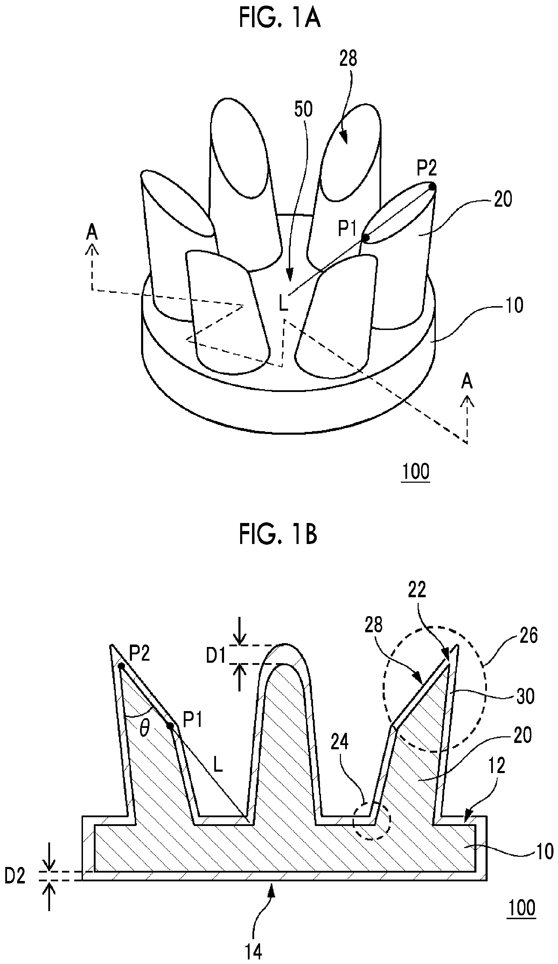

Figs. 1A and 1B are schematic diagrams showing the summary of a biomedical electrode 100 according to the embodiment, in which Fig. 1A is a perspective view and Fig. 1B is a cross-sectional view taken along line A-A of Fig. 1A.

As shown in Figs. 1A and 1B, the biomedical electrode 100 includes a plate-shaped support portion 10, pillar portions (elastic pillar portions) 20, and a conductive resin layer 30.

The plate-shaped support portion 10 is formed of an insulating elastic member, in which the plurality of pillar portions 20 are provided on a first surface 12.

The plurality of pillar portions 20 are formed of an insulating elastic member and are configured to protrude from the first surface 12.

The conductive resin layer 30 is formed of a conductive elastic member and may be formed to cover at least a surface of a distal end 22 (a part of a distal end portion 26) of the pillar portion 20.

When the distal end portion 26 of the biomedical electrode 100 comes into contact with a measurement target, a bioelectric signal detected by the pillar portions 20 through the conductive resin layer 30 can be transmitted to an external connection portion 110 (connector) provided in the plate-shaped support portion 10. The bioelectric signal detected by the biomedical electrode 100 can be transmitted to an external apparatus through the connector.

The shape of the plate-shaped support portion 10 in a top view may be, for example, a substantially circular shape such as an elliptical shape or a perfect circular shape or may be a substantially polygonal shape such as a square shape, a rectangular shape, a pentagonal shape, or a hexagonal shape. Roundness (R) may be imparted to a corner portion of the polygonal shape.

Here, the top view represents being observed from the top when seen from the distal end 22 of the pillar portion 20 toward the plate-shaped support portion 10.

The first surface 12 of the plate-shaped support portion 10 may be configured to be flat or have a curved surface in a part or the entirety thereof.

Here, the first surface 12 may be configured with a surface that passes through at least three contact points in contact with a side surface of the pillar portion 20 and the plate-shaped support portion 10. A direction perpendicular to the first surface 12 may be configured to be parallel to the pressing direction.

In addition, the plate-shaped support portion 10 and the pillar portion 20 may be seamlessly configured without an interface therebetween.

A second surface 14 of the plate-shaped support portion 10 may have a structure that can be connected to the connector. For example, an electrode that can be electrically connected to the connector may be buried in the second surface 14 opposite to the first surface 12 in a state where a part of the second surface 14 is exposed.

In addition, at least a part or the entirety of the second surface 14 may be covered with a conductive elastic member. At this time, a side surface of the plate-shaped support portion 10 may not be covered with a conductive elastic member.

The plate-shaped support portion 10 may be integrally formed of the same resin material as the plurality of pillar portions 20. For example, by molding a curable elastomer composition such as a silicone rubber-based curable composition described below, the plate-shaped support portion 10 and the plurality of pillar portions 20 may be seamlessly bonded to each other to obtain a molded body. As a result, an elastic molded body having excellent flexibility and strength can be realized.

Each of the plate-shaped support portion 10 and the pillar portions 20 may include a conductive filler and, from the viewpoint of costs, may be formed of an insulating silicone rubber (rubber molded body) including a silicone rubber without including a conductive filler as an insulating elastic member.

It is preferable that the plurality of pillar portions 20 are arranged to surround a center portion 50 of the first surface 12 of the plate-shaped support portion 10. In other words, the plurality of pillar portions 20 are arranged along an outer peripheral edge of the plate-shaped support portion 10. As a result, the followability of the pillar portion 20 to a living body can be improved.

In a case where the shape of the first surface 12 of the plate-shaped support portion 10 in a top view is a substantially circular shape, the plurality of pillar portions 20 may be arranged on the first surface 12 in a substantially circular shape or a substantially elliptical shape. The pillar portions 20 may be arranged to configure one or more concentric circles around the center portion 50. As a result, when coming into contact with a measurement target, the distal end portion 26 of the pillar portion 20 substantially uniformly spreads in a radial direction. Therefore, the measurement stability can be improved.

The center portion 50 of the plate-shaped support portion 10 can be positioned at substantially equal distances from the distal ends 22 of the plurality of pillar portions 20 present on the same circumference.

The pillar portion 20 may be configured in a substantially truncated conical shape or a truncated pyramid shape. In particular, from the viewpoint of measurement stability, a substantially truncated conical shape is preferable. The truncated conical pillar portion 20 is configured such that the diameter decreases from a connection portion (base end portion 24) to the plate-shaped support portion 10 toward the distal end 22 side. Therefore, the manufacturing stability during molding can be improved. In addition, an inner side surface of the pillar portion 20 has a tapered shape that spreads from the inside toward the outside when seen in the pressing direction. Therefore, a variation in the deformation of the pillar portion 20 can be further suppressed.

The pillar portion 20 may have a structure in which the center is eccentric with respect to the direction perpendicular to the first surface 12 of the plate-shaped support portion 10. From the viewpoint of manufacturing stability, it is preferable that the central axis of the pillar portion 20 having the eccentric structure is inclined from the center portion 50 of the plate-shaped support portion 10 toward the outside.

The inclination of the central axis of the pillar portion 20 refers to an outside angle (acute angle) between the central axis of the pillar portion 20 and the first surface 12 (surface) of the plate-shaped support portion 10 in a cross-sectional view passing through the center portion 50 and the center portion of the pillar portion 20 from the inside toward the outside of each of the pillar portions 20 with respect to the center portion 50.

The inclination of the central axis of the pillar portion 20 is, for example, 45 degrees to 90 degrees, preferably 50 degrees to 88 degrees, and more preferably 60 degrees to 85 degrees. In the above-described numerical range, releasability from a mold can be improved.

In the biomedical electrode 100, as shown in Figs. 1A and 1B, in an inclined surface 28 of the pillar portion 20, a point closest to the first surface 12 is represented by P1, a point farthest from the first surface 12 is represented by P2, and a half line extending from P2 to P1 is represented by L.

The first surface 12 may be configured with a surface that passes through at least three contact points in contact with the side surface of the pillar portion 20 and the plate-shaped support portion 10. This contact point may be a contact point between the base end portion 24 positioned on the side surface of the pillar portion 20 and the plate-shaped support portion 10.

The center portion 50 may be a region including a center of gravity position of the first surface 12 when seen from the direction perpendicular to the first surface 12. In addition, when the shortest distance from the center of gravity position of the first surface 12 to a circumference of the first surface 12 is represented by Dmin, the center portion 50 is preferably 2/10 Dmin or less from the center of gravity position and may be 1/10 Dmin or less from the center of gravity position. The vicinity of the center portion 50 is, for example, 5/10 Dmin or less from the center of gravity position, preferably 4/10 Dmin from the center of gravity position, and more preferably 3/10 Dmin or less from the center of gravity position.

In the plurality of pillar portions 20, the point P1 is configured to be present at a position closer to the center portion 50 than the point P2 when seen from the direction perpendicular to the first surface 12. With the above-described configuration, the plurality of pillar portions 20 are arranged to surround the center portion 50 of the first surface 12 of the plate-shaped support portion 10. In a case where a plurality of points P1 and a plurality of points P2 are present, two points having the shortest distance of a line segment connecting P1 and P2 may be adopted.

The half line L is configured to pass through the center portion 50 or the vicinity of the center portion 50 in the plurality of pillar portions 20. With the above-described configuration, the distal end portions 26 of the plurality of 20 has the inclined surface 28 that is configured such that the height of the pillar portion 20 on the outside is higher than that on the inside in a cross-sectional view in a direction passing through the outside from the inside of each of the pillar portions 20, for example, in a radial direction. That is, a perpendicular line of the inclined surface 28 of each of the pillar portions 20 may be configured to face the center portion 50 of the plate-shaped support portion 10.

The inclined surface 28 of the plurality of pillar portions 20 is configured to be inclined at a predetermined angle with respect to the first surface 12 without being parallel thereto, that is, is configured to form an acute angle. For example, the angle between the inclined surface 28 and the first surface 12 may be more than 0 degrees and less than 90 degrees, may be 10 degrees to 75 degrees, may be 30 degrees to 70 degrees, or may be 40 degrees to 65 degrees. In the above-described numerical range, the followability of the inclined surface 28 can be improved.

When the above-described inclined surface 28 comes into contact with a measurement target, the inclined surface 28 can favorably follow the measurement surface of the measurement target. Therefore, all the plurality of pillar portions 20 are deformed to spread from the inside toward the outside, and a variation in the deformation directions can be suppressed. As a result, a variation in the contact area of the inclined surface 28 can be suppressed. Accordingly, the measurement stability in the biomedical electrode 100 can be improved.

The half lines L may be configured to intersect each other in the center portion 50 in the plurality of pillar portions 20. In addition, at least two or more of the plurality of pillar portions 20 may be configured to intersect each other at the center of gravity position of the first surface 12. As a result, a variation in the deformation direction of the pillar portion 20 can be further suppressed.

The distal end portion 26 of the pillar portion 20 may be configured not to have a flat surface parallel to the first surface 12. As a result, the followability of the inclined surface 28 can be improved.

The inclined surface 28 of the pillar portion 20 and the side surface of the pillar portion 20 may be configured to intersect each other through the point P1. With the above-described configuration, the area of the inclined surface 28 can be controlled to be in an appropriate range. As a result, the followability of the inclined surface 28 to the measurement surface can be improved.

When seen from the distal end side toward the base end side of the pillar portion 20, the shape of the inclined surface 28 in a top view can be made to be a substantially elliptical shape. This substantially elliptical shape may have a major axis of in a radial direction of a substantially circumference where the pillar portion 20 is arranged. The followability to the measurement surface can be improved.

As shown in Fig. 1B, in a cross-sectional view passing through the outside from the inside of each of the pillar portions 20, specifically, in a cross-section passing through the points P1 and P2, an inclination angle θ of the inclined surface 28 refers to an angle between an outside surface of the pillar portion 20 and the inclined surface 28.

The inclination angle θ of the inclined surface 28 is, for example, 10 to 89 degrees, preferably 15 degrees to 80 degrees, more preferably 20 degrees to 60 degrees, and still more preferably 25 degrees to 50 degrees. By adjusting the inclination angle θ of the inclined surface 28 to be the lower limit value or more, the followability to the measurement surface can be improved. By adjusting the inclination angle θ of the inclined surface 28 to be the upper limit value or less, a variation in the deformed state can be suppressed.

Roundness (R) may be imparted to a corner portion formed at a position where the inclined surface 28 of the pillar portion 20 and the side surface of pillar portion 20 intersect each other. In particular, by imparting roundness to the corner portion of the inclined surface 28 and the outside surface, discomfort during the contact with a measurement target can be suppressed, and a variation in the deformation state of the pillar portion 20 can be further suppressed.

In a cross-sectional view passing through the center portion 50 and the pillar portion 20, the length of the pillar portion 20 from the base end portion 24 connected to the plate-shaped support portion 10 to the distal end 22 can be configured to be longer than the width of the pillar portion in the base end portion 24. As a result, the arrangement density of the pillar portions 20 can be improved. In addition, the pillar portions 20 can be suppressed from coming into contact with each other during deformation.

The conductive resin layer 30 may have any configuration as long as it can be electrically connected to the connector connected to the second surface 14 of the plate-shaped support portion 10.

The conductive resin layer 30 may be configured to cover the entirety of at least the surface of the distal end 22 of the pillar portion 20, to cover the entirety of a surface from the distal end 22 of the pillar portion 20 to a middle portion of the base end portion 24, or to cover the entirety of the surface of the pillar portion 20. In this case, the biomedical electrode 100 includes a conductive line that is electrically connected to the conductive resin layer 30 and is arranged inside the pillar portion 20 from the distal end side toward the base end side. A material or an arrangement position of the conductive line is not particularly limited as long as the conductive line is conductive. As a result, in the biomedical electrode 100, the bioelectrical potential can be measured through the conductive line.

Alternatively, the conductive resin layer 30 may be configured to cover the first surface 12 or the second surface 14 of the plate-shaped support portion 10, or to cover the surface of the plate-shaped support portion 10 and the surface of the pillar portion 20. In this case, the conductive resin layer 30 may be configured to cover at least the surface of the plate-shaped support portion 10 on the first surface 12 side continuously from the surface of the pillar portion 20 or may be configured to cover a part of the first surface 12 or the second surface 14. As a result, in the biomedical electrode 100, the bioelectrical potential can be measured through the conductive resin layer 30.

The conductive resin layer 30 is formed of a conductive silicone rubber including a conductive filler and a silicone rubber as a conductive elastic member. For example, a conductive solution (conductive silicone rubber-based curable composition) in which a conductive filler is added to an insulating silicone rubber-based curable composition not including a conductive filler described below is applied to the above-described molded body such that the conductive resin layer 30 can be formed. By using the same silicone rubber material as the silicone rubber forming the plate-shaped support portion 10 or the pillar portion 20, the adhesion of the conductive resin layer 30 can be improved.

As the conductive filler, a well-known conductive material may be used. The conductive filler may include one or more selected from the group consisting of metal particles, silver or silver chloride particles, metal fiber, metal-coated fiber, carbon black, acetylene black, graphite, carbon fiber, carbon nanotube, a conductive polymer, conductive polymer-coated fiber, and metal nanowire.

The metal forming the conductive filler is not particularly limited. For example, the metal may include at least one or two or more among copper, silver, gold, nickel, tin, lead, zinc, bismuth, antimony, silver or silver chloride, and alloys thereof. In particular, silver or copper is preferable from the viewpoint of high conductivity or high availability.

The lower limit value of the content of the conductive filler is, for example, 30 mass% or higher, preferably 35 mass% or higher, and more preferably 40 mass% or higher with respect to 100 mass% of the silicone rubber in the conductive resin layer 30. As a result, even in the case of a thin film, the transmission performance of a bioelectric signal can be improved. On the other hand, the upper limit value of the content of the conductive filler is, for example, 90 mass% or lower, preferably 85 mass% or lower and more preferably 80 mass% or lower with respect to 100 mass% of the silicone rubber in the conductive resin layer 30. As a result, the durability of the conductive resin layer 30 for the deformation of the pillar portion 20 can be improved.

The lower limit value of the thickness of the conductive resin layer 30 is, for example, 5 µm or more, preferably 8 µm or more, and more preferably 10 µm or more. As a result, the durability during repeated use can be improved. On the other hand, the upper limit value of the thickness of the conductive resin layer 30 is, for example, 200 µm or less, preferably 150 µm or less, more preferably 100 µm or less, and still more preferably 50 µm or less. As a result, the deformation easiness of the pillar portion 20 can be maintained. In a cross-sectional view of the pillar portion 20, the thickness of the conductive resin layer 30 on at least a part of the distal end 22 or the side surface of the pillar portion 20 is preferably in the numerical range.

The thickness of the conductive resin layer 30 positioned at the distal end 22 of the pillar portion 20 may in the above-described range.

Regarding the thickness of the conductive resin layer 30, a thickness D1 on the surface of the distal end 22 of the pillar portion 20 may be configured to be thicker than a thickness D2 on the second surface 14 of the plate-shaped support portion 10. For example, after applying the above-described conductive solution, a part of the pillar portion 20 coated with the conductive resin layer 30 may be dipped (dip coating) in a paste-like conductive solution. As a result, while reducing the thickness of the entire conductive resin layer 30, the thickness of the distal end 22 of the pillar portion 20 or the thickness of a portion from the distal end 22 to a predetermined portion (for example, 1/2, 1/3, or 1/4 of the entire pillar portion 20) can be made to be relatively thick. It is preferable that this thick region is provided in the entirety of the distal end portion of the pillar portion 20 in the circumferential direction. As a result, peeling of the conductive resin layer 30 in the distal end portion can be suppressed, and damages such as disconnection of the pillar portion 20 can be suppressed. Therefore, the durability of the biomedical electrode 100 can be improved.

Here, the silicone rubber-based curable composition will be described.

The silicone rubber can be formed of a cured product of the silicone rubber-based curable composition. A step of curing the silicone rubber-based curable resin composition is performed by heating (primary curing) the composition, for example, at 100°C to 250°C for 1 minute to 30 minutes and post-baking (secondary curing) the heated composition at 200°C for 1 hour to 4 hours.

The silicone rubber-based curable composition according to the embodiment may include a vinyl group-containing organopolysiloxane (A) . The vinyl group-containing organopolysiloxane (A) is a polymer including the silicone rubber-based curable composition according to the embodiment as a main component.

The vinyl group-containing organopolysiloxane (A) may include a vinyl group-containing linear organopolysiloxane (A1) having a linear structure.

The vinyl group-containing linear organopolysiloxane (A1) has a linear structure and includes a vinyl group, in which the vinyl group functions as a crosslinking point during curing.

The content of the vinyl group in the vinyl group-containing linear organopolysiloxane (A1) is not particularly limited. For example, the vinyl group-containing linear organopolysiloxane (A1) includes two or more vinyl groups in the molecule, and the content thereof is preferably 15 mol% or lower and more preferably 0.01% to 12 mol%. As a result, the content of the vinyl group in the vinyl group-containing linear organopolysiloxane (A1) can be optimized, and a network between the respective components can be reliably formed. In the embodiment, a range represented by "to" includes numerical values of both ends.

In this specification, the content of the vinyl group represents mol% of a vinyl group-containing siloxane unit with respect to 100 mol% of all the units forming the vinyl group-containing linear organopolysiloxane (A1). In this case, it is assumed that one vinyl group is present for each vinyl group-containing siloxane unit.

In addition, the polymerization degree of the vinyl group-containing linear organopolysiloxane (A1) is not particularly limited and is, for example, preferably in a range of about 1000 to 10000 and more preferably in a range of about 2000 to 5000. The polymerization degree can be obtained as, for example, a number average polymerization degree (or number average molecular weight) in terms of polystyrene in GPC (gel permeation chromatography) in which chloroform is used as an eluent.

Further, the specific gravity of the vinyl group-containing linear organopolysiloxane (A1) is not particularly limited and is preferably in a range of about 0.9 to 1.1.

By using the vinyl group-containing linear organopolysiloxane (A1) having the polymerization degree and the specific gravity in the above-described ranges, the heat resistance, flame retardancy, chemical stability, and the like of the obtained silicone rubber can be improved.

It is preferable that the vinyl group-containing linear organopolysiloxane (A1) has a structure represented by the following Formula (1).

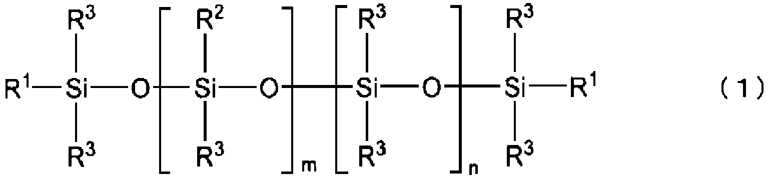

In Formula (1), R¹ represents a substituted or unsubstituted alkyl group, alkenyl group, or aryl group having 1 to 10 carbon atoms, or a hydrocarbon group including a combination thereof. Examples of the alkyl group having 1 to 10 carbon atoms include a methyl group, an ethyl group, and a propyl group. In particular, a methyl group is preferable. Examples of the alkenyl group having 1 to 10 carbon atoms include a vinyl group, an allyl group, and a butenyl group. In particular, a vinyl group is preferable. Examples of the aryl group having 1 to 10 carbon atoms include a phenyl group.

In addition, R² represents a substituted or unsubstituted alkyl group, alkenyl group, or aryl group having 1 to 10 carbon atoms, or a hydrocarbon group including a combination thereof. Examples of the alkyl group having 1 to 10 carbon atoms include a methyl group, an ethyl group, and a propyl group. In particular, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In addition, R³ represents a substituted or unsubstituted alkyl group or aryl group having 1 to 8 carbon atoms, or a hydrocarbon group including a combination thereof. Examples of the alkyl group having 1 to 8 carbon atoms include a methyl group, an ethyl group, and a propyl group. In particular, a methyl group is preferable. Examples of the aryl group having 1 to 8 carbon atoms include a phenyl group.

Further, examples of a substituent of R¹ and R² in Formula (1) include a methyl group and a vinyl group. Examples of a substituent of R³ include a methyl group.

In Formula (1), a plurality of R¹'s may be independent from each other and may be the same as or different from each other. Further, the same can be applied to R² and R³.

Further, m and n each independently represent the number of repeating units forming the vinyl group-containing linear organopolysiloxane (A1) represented by Formula (1), m represents an integer of 0 to 2000, and n represents an integer of 1000 to 10000. m represents preferably 0 to 1000, and n represents preferably 2000 to 5000.

In addition, examples of a specific structure of the vinyl group-containing linear organopolysiloxane (A1) represented by Formula (1) include a structure represented by the following Formula (1-1).

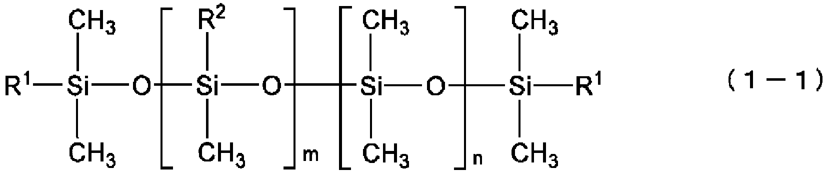

In Formula (1-1), R¹ and R² each independently represent a methyl group or a vinyl group, and at least one of R¹ or R² represents a vinyl group.

Further, it is preferable that as the vinyl group-containing linear organopolysiloxane (A1), a first vinyl group-containing linear organopolysiloxane (A1-1) having two or more vinyl groups in the molecule in which the vinyl group content is 0.4 mol% or lower; or a second vinyl group-containing linear organopolysiloxane (A1-2) in which the vinyl group content is 0.5 to 15 mol% is included. By using the first vinyl group-containing linear organopolysiloxane (A1-1) having the general vinyl group content and the second vinyl group-containing linear organopolysiloxane (A1-2) having the higher vinyl group in combination as raw rubber that is a raw material of the silicone rubber, the vinyl groups can be distributed, and a structure of the crosslinking density in the crosslinked network of the silicone rubber can be more effectively formed. As a result, the tear strength of the silicone rubber can be more effectively improved.

Specifically, as the vinyl group-containing linear organopolysiloxane (A1), for example, the first vinyl group-containing linear organopolysiloxane (A1-1) having two or more of a unit in which R¹ in Formula (1-1) represents a vinyl group and/or a unit in which R² in Formula (1-1) represents a vinyl group in the molecule and the content is 0.4 mol% or lower, or the second vinyl group-containing linear organopolysiloxane (A1-2) including 0.5 to 15 mol% of a unit in which R¹ in Formula (1-1) represents a vinyl group and/or a unit in which R² in Formula (1-1) represents a vinyl group is preferably used.

In addition, in the first vinyl group-containing linear organopolysiloxane (A1-1), the vinyl group content is preferably 0.01 to 0.2 mol%. In addition, in the second vinyl group-containing linear organopolysiloxane (A1-2) , the vinyl group content is preferably 0.8 to 12 mol%.

Further, in a case where the first vinyl group-containing linear organopolysiloxane (A1-1) and the second vinyl group-containing linear organopolysiloxane (A1-2) are mixed in combination, the ratio between (A1-1) and (A1-2) is not particularly limited. For example, the weight ratio (A1-1) : (A1-2) is preferably 50:50 to 95:5 and more preferably 80:20 to 90:10.

As each of the first and second vinyl group-containing linear organopolysiloxanes (A1-1) and (A1-2), only one kind may be used, or two or more kinds may be used in combination.

In addition, the vinyl group-containing organopolysiloxane (A) may include a vinyl group-containing branched organopolysiloxane (A2) having a branched structure.

### <<Organohydrogen Polysiloxane (B)>>

The silicone rubber-based curable composition according to the embodiment may include an organohydrogen polysiloxane (B).

The organohydrogen polysiloxane (B) is classified into a linear organohydrogen polysiloxane (B1) having a linear structure and a branched organohydrogen polysiloxane (B2) having a branched structure and may include either or both of (B1) and (B2).

The linear organohydrogen polysiloxane (B1) is a polymer that has a linear structure and a structure (≡Si-H) in which hydrogen is directly bonded to Si and that is obtained by a hydrosilylation reaction of the vinyl group in the vinyl group-containing organopolysiloxane (A) and a vinyl group in a component mixed in the silicone rubber-based curable composition to crosslink the components.

The molecular weight of the linear organohydrogen polysiloxane (B1) is not particularly limited. For example, the weight average molecular weight is preferably 20000 or lower and more preferably 1000 or higher and 10000 or lower.

The weight average molecular weight of the linear organohydrogen polysiloxane (B1) can be measured in terms of polystyrene in GPC (gel permeation chromatography) in which chloroform is used as an eluent.

In addition, it is preferable that, typically, the linear organohydrogen polysiloxane (B1) does not have a vinyl group. As a result, the crosslinking reaction in the molecule of the linear organohydrogen polysiloxane (B1) can be reliably prevented from progressing.

It is preferable to use the linear organohydrogen polysiloxane (B1), for example, having a structure represented by the following Formula (2).

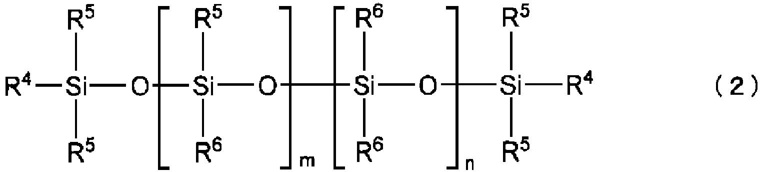

In Formula (2), R⁴ represents a substituted or unsubstituted alkyl group, alkenyl group, or aryl group having 1 to 10 carbon atoms, a hydrocarbon group including a combination thereof, or a hydride group. Examples of the alkyl group having 1 to 10 carbon atoms include a methyl group, an ethyl group, and a propyl group. In particular, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In addition, R⁵ represents a substituted or unsubstituted alkyl group, alkenyl group, or aryl group having 1 to 10 carbon atoms, a hydrocarbon group including a combination thereof, or a hydride group. Examples of the alkyl group having 1 to 10 carbon atoms include a methyl group, an ethyl group, and a propyl group. In particular, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In Formula (2), a plurality of R⁴'s may be independent from each other and may be the same as or different from each other. The same can be applied to R⁵. In this case, at least two or more among a plurality of R⁴'s and a plurality of R⁵'s represent a hydride group.

In addition, R⁶ represents a substituted or unsubstituted alkyl group or aryl group having 1 to 8 carbon atoms, or a hydrocarbon group including a combination thereof. Examples of the alkyl group having 1 to 8 carbon atoms include a methyl group, an ethyl group, and a propyl group. In particular, a methyl group is preferable. Examples of the aryl group having 1 to 8 carbon atoms include a phenyl group. A plurality of R⁶'s may be independent from each other and may be the same as or different from each other.

Examples of a substituent of R⁴, R⁵, and R⁶ in Formula (2) include a methyl group and a vinyl group. From the viewpoint of preventing a crosslinking reaction in the molecule, a methyl group is preferable.

Further, m and n each independently represent the number of repeating units forming the linear organohydrogen polysiloxane (B1) represented by Formula (2), m represents an integer of 2 to 150, and n represents an integer of 2 to 150. It is preferable that m represents an integer of 2 to 100 and n represents an integer of 2 to 100.

As the linear organohydrogen polysiloxane (B1), only one kind may be used alone, or two or more kinds may be used in combination.

The branched organohydrogen polysiloxane (B2) has a branched structure and thus is a component that largely contributes to the formation of a structure of the crosslinking density in the silicone rubber system by forming a region having a high crosslinking density. In addition, as in the linear organohydrogen polysiloxane (B1), the branched organohydrogen polysiloxane (B2) is a polymer that a structure (≡Si-H) in which hydrogen is directly bonded to Si and that is obtained by a hydrosilylation reaction of the vinyl group in the vinyl group-containing organopolysiloxane (A) and a vinyl group in a component mixed in the silicone rubber-based curable composition to crosslink the components.

In addition, the specific gravity of the branched organohydrogen polysiloxane (B2) is in a range of 0.9 to 0.95.

Further, it is preferable that, typically, the branched organohydrogen polysiloxane (B2) does not have a vinyl group. As a result, the crosslinking reaction in the molecule of the branched organohydrogen polysiloxane (B2) can be reliably prevented from progressing.

In addition, it is preferable that the branched organohydrogen polysiloxane (B2) is represented by Average Compositional Formula (c) described below.

Average Compositional Formula (c) (Hₐ(R⁷)₃₋ₐSiO_{1/2})ₘ(SiO_{4/2})ₙ

(In Formula (c), R⁷ represents a monovalent organic group, a represents an integer of 1 to 3, m represents the number of Hₐ(R⁷)₃₋ₐSiO_{1/2} units, and n represents the number of SiO_{4/2} units.)

In Formula (c), R⁷ represents a monovalent organic group, and represents preferably a substituted or unsubstituted alkyl group or aryl group having 1 to 10 carbon atoms, or a hydrocarbon group including a combination thereof. Examples of the alkyl group having 1 to 10 carbon atoms include a methyl group, an ethyl group, and a propyl group. In particular, a methyl group is preferable. Examples of the aryl group having 1 to 10 carbon atoms include a phenyl group.

In Formula (c), a represents the number of hydride groups (hydrogen atoms directly bonded to Si) which is an integer of 1 to 3 and preferably 1.

In addition, in Formula (c), m represents the number of Hₐ(R⁷)₃₋ₐSiO_{1/2} units, and n represents the number of SiO_{4/2} units.

The branched organohydrogen polysiloxane (B2) has a branched structure. The linear organohydrogen polysiloxane (B1) and the branched organohydrogen polysiloxane (B2) are different from each other in that whether the structure is linear or branched. A ratio of the number (R/Si) of alkyl groups R bonded to Si to the number of Si that is 1 is 1.8 to 2.1 in the linear organohydrogen polysiloxane (B1) and is 0.8 to 1.7 in the branched organohydrogen polysiloxane (B2) .

Since the branched organohydrogen polysiloxane (B2) has a branched structure, the amount of residues is 5% or higher, for example, during heating to 1000°C at a temperature increase rate of 10 °C/min in a nitrogen atmosphere. On the other hand, since the linear organohydrogen polysiloxane (B1) is linear, the amount of residues after heating under the above-described conditions is substantially zero.

In addition, specific examples of the branched organohydrogen polysiloxane (B2) include a branched organohydrogen polysiloxane (B2) having a structure represented by the following Formula (3).

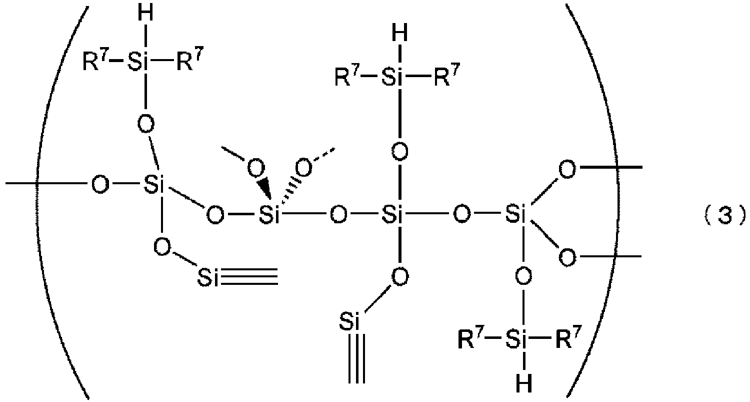

In Formula (3), R⁷ represents a substituted or unsubstituted alkyl group or aryl group having 1 to 8 carbon atoms, a hydrocarbon group including a combination thereof, or a hydrogen atom. Examples of the alkyl group having 1 to 8 carbon atoms include a methyl group, an ethyl group, and a propyl group. In particular, a methyl group is preferable. Examples of the aryl group having 1 to 8 carbon atoms include a phenyl group. Examples of a substituent of R⁷ include a methyl group.

In Formula (3), a plurality of R⁷'s may be independent from each other and may be the same as or different from each other.

In addition, in Formula (3), "-O-Si≡" represents that Si has a branched structure that spreads three-dimensionally.

As the branched organohydrogen polysiloxane (B2), only one kind may be used alone, or two or more kinds may be used in combination.

In addition, in each of the linear organohydrogen polysiloxane (B1) and the branched organohydrogen polysiloxane (B2), the amount of hydrogen atoms (hydride groups) directly bonded to Si is not particularly limited. In the silicone rubber-based curable composition, the total amount of hydride groups in the linear organohydrogen polysiloxane (B1) and the branched organohydrogen polysiloxane (B2) is preferably 0.5 to 5 mol and more preferably 1 to 3.5 mol with respect to 1 mol of vinyl group in the vinyl group-containing linear organopolysiloxane (A1). As a result, a crosslinked network can be reliably formed between the linear organohydrogen polysiloxane (B1) and the branched organohydrogen polysiloxane (B2), and the vinyl group-containing linear organopolysiloxane (A1).

### <<Silica Particles (C)>>

The silicone rubber-based curable composition according to the embodiment optionally includes silica particles (C). As a result, the hardness or mechanical strength of the elastomer can be improved.

The silica particles (C) are not particularly limited. For example, fumed silica, pyrogenic silica, or precipitated silica is used. As the silica particles (C), one kind may be used alone, or two or more kinds may be used in combination.

The specific surface area of the silica particles (C) measured using, for example, a BET method is, for example, preferably 50 to 400 m²/g and more preferably 100 to 400 m²/g. In addition, the average primary particle size of the silica particles (C) is, for example, preferably 1 to 100 nm and more preferably about 5 to 20 nm.

By using the silica particles (C) having the specific surface area and the average particle size in the above-described range, the hardness and the mechanical strength, in particular, the tensile strength of the formed silicone rubber can be improved.

### <<Silane Coupling Agent (D)>

The silicone rubber-based curable composition according to the embodiment may include a silane coupling agent (D).

The silane coupling agent (D) may include a hydrolyzable group. The hydrolyzable group is hydrolyzed into a hydroxyl group by water, and this hydroxyl group reacts with a hydroxyl group on the silica particles (C) in a dehydration synthesis reaction. As a result, the surfaces of the silica particles (C) can be modified.

In addition, the silane coupling agent (D) may include a silane coupling agent having a hydrophobic group. As a result, this hydrophobic group is added to the surfaces of the silica particles (C). Therefore, it is presumed, in the silicone rubber-based curable composition and the silicone rubber, the cohesion force between the silica particles (C) decreases (cohesion through a hydrogen bond formed by a silanol group decreases), and thus the dispersibility of the silica particles (C) in the silicone rubber-based curable composition is improved. As a result, the area of an interface between the silica particles (C) and a rubber matrix increases, and a reinforcing effect of the silica particles (C) increases. Further, it is presumed that, when the rubber matrix is deformed, the slipperiness of the silica particles (C) in the matrix is improved. By improving the dispersibility and slipperiness of the silica particles (C), the mechanical strength (for example, tensile strength or tear strength) of the silicone rubber by the silica particles (C) is improved.

Further, the silane coupling agent (D) may include a silane coupling agent having a vinyl group. As a result, a vinyl group is introduced into the surface of the silica particles (C). Therefore, when the silicone rubber-based curable composition is cured, that is, when a vinyl group in the vinyl group-containing organopolysiloxane (A) and a hydride group in the organohydrogen polysiloxane (B) react with each other in a hydrosilylation reaction such that a network (crosslinked structure) is formed, a vinyl group in the silica particles (C) gets involved with the hydrosilylation reaction with the hydride group in the organohydrogen polysiloxane (B). Therefore, the silica particles (C) are also incorporated into the network. As a result, low hardness and high modulus of the formed silicone rubber can be realized.

As the silane coupling agent (D), the silane coupling agent having a hydrophobic group and the silane coupling agent having a vinyl group can be used in combination.

Examples of the silane coupling agent (D) include a silane coupling agent represented by the following Formula (4).

Yₙ-Si-(X)₄₋ₙ... (4)

In Formula (4), n represents an integer of 1 to 3. Y represents any functional group of a hydrophobic group, a hydrophilic group, or a vinyl group, when n represents 1, Y represents a hydrophobic group, and when n represents 2 or 3, at least one of Y's represents a hydrophobic group. X represents a hydrolyzable group.

The hydrophobic group is an alkyl group or aryl group having 1 to 6 carbon atoms, or a hydrocarbon group including a combination thereof. Examples of the hydrophobic group include a methyl group, an ethyl group, a propyl group, and a phenyl group. In particular, a methyl group is preferable.

In addition, examples of the hydrophilic group include a hydroxyl group, a sulfonate group, a carboxyl group, and a carbonyl group. In particular, a hydroxyl group is preferable. The hydrophilic group may be included as a functional group. However, it is preferable that the hydrophilic group is not included from the viewpoint of imparting hydrophobicity to the silane coupling agent (D) .

Further, examples of the hydrolyzable group include an alkoxy group such as a methoxy group or an ethoxy group, a chloro group, and a silazane group. In particular, a silazane group is preferable from the viewpoint of high reactivity with the silica particles (C) . The silane coupling agent having a silazane group as the hydrolyzable group includes two structures represented by (Yₙ-Si-) in Formula (4) due to properties of the structure.

Specific examples of the silane coupling agent (D) represented by Formula (4) are as follows.

Examples of the silane coupling agent having a hydrophobic group as the functional group include: an alkoxysilane such as methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, or decyltrimethoxysilane; a chlorosilane such as methyltrichlorosilane, dimethyldichlorosilane, trimethylchlorosilane, or phenyltrichlorosilane; and hexamethyldisilazane. In particular, a silane coupling agent having a trimethylsilyl group that includes one or more selected from the group consisting of hexamethyldisilazane, trimethylchlorosilane, trimethylmethoxysilane, and trimethylethoxysilane is preferable.

Examples of the silane coupling agent having a vinyl group as the functional group include: an alkoxysilane such as methacryloxypropyltriethoxysilane, methacryloxypropyltrimethoxysilane, methacryloxypropylmethyldiethoxysilane, methacryloxypropylmethyldimethoxysilane, vinyltriethoxysilane, vinyltrimethoxysilane, or vinylmethyldimethoxysilane; a chlorosilane such as vinyltrichlorosilane or vinylmethyldichlorosilane; and divinyltetramethyldisilazane. In particular, a silane coupling agent having a vinyl group-containing organosilyl group that includes one or more selected from the group consisting of methacryloxypropyltriethoxysilane, methacryloxypropyltrimethoxysilane, methacryloxypropylmethyldiethoxysilane, methacryloxypropylmethyldimethoxysilane, divinyltetramethyldisilazane, vinyltriethoxysilane, vinyltrimethoxysilane, and vinylmethyldimethoxysilane is preferable.

In addition, in a case where the silane coupling agent (D) includes two kinds including a silane coupling agent having a trimethylsilyl group and a silane coupling agent having a vinyl group-containing organosilyl group, it is preferable that hexamethyldisilazane is included as the silane coupling agent having a hydrophobic group and divinyltetramethyldisilazane is included as the silane coupling agent having a vinyl group.

In a case where the silane coupling agent (D1) having a trimethylsilyl group and the silane coupling agent (D2) having a vinyl group-containing organosilyl group are used in combination, a ratio between (D1) and (D2) is not particularly limited. For example, a weight ratio (D1):(D2) is 1:0.001 to 1:0.35, preferably 1:0.01 to 1:0.20, and more preferably 1:0.03 to 1:0.15. In the above-described numerical range, desired physical properties of the silicone rubber can be obtained. Specifically, a balance between the dispersibility of silica in the rubber and the crosslinkability of the rubber can be realized.

In the embodiment, the lower limit value of the content of the silane coupling agent (D) is preferably 1 mass% or higher, more preferably 3 mass% or higher, and still more preferably 5 mass% or higher with respect to 100 parts by weight of the total amount of the vinyl group-containing organopolysiloxane (A). In addition, the upper limit value of the content of the silane coupling agent (D) is preferably 100 mass% or lower, more preferably 80 mass% or lower, and still more preferably 40 mass% or lower with respect to 100 parts by weight of the total amount of the vinyl group-containing organopolysiloxane (A).

By adjusting the content of the silane coupling agent (D) to be the lower limit value or higher, the adhesion between the pillar portion including the elastomer and the conductive resin layer can be improved. In addition, the improvement of the mechanical strength of the silicone rubber can be promoted. In addition, by adjusting the content of the silane coupling agent (D) to be the upper limit value or lower, the silicone rubber can be made to have appropriate mechanical properties.

### <<Platinum or Platinum Compound (E)>>

The silicone rubber-based curable composition according to the embodiment may include platinum or platinum compound (E).

The platinum or platinum compound (E) is a catalyst component that functions as a catalyst during curing. The addition amount of the platinum or platinum compound (E) is the amount of the catalyst.

As the platinum or platinum compound (E), a well-known compound can be used, and examples thereof include platinum black, silica or carbon black on which platinum is supported, chloroplatinic acid or an alcohol solution of chloroplatinic acid, a complex salt of chloroplatinic acid and olefin, and a complex salt of chloroplatinic acid and vinylsilxoane.

As the platinum or platinum compound (E), only one kind may be used alone, or two or more kinds may be used in combination.

In the embodiment, the content of the platinum or platinum compound (E) in the silicone rubber-based curable composition refers to the amount of the catalyst and can be appropriately set. Specifically, the content of platinum group metal by weight is 0.01 to 1000 ppm and preferably 0.1 to 500 ppm with respect to 100 parts by weight of the vinyl group-containing organopolysiloxane (A), the silica particles (C), and the silane coupling agent (D).

By adjusting the content of the platinum or platinum compound (E) to be the lower limit value or higher, the silicone rubber-based curable composition can be cured at an appropriate rate. In addition, by adjusting the content of the platinum or platinum compound (E) to be the upper limit value or lower, a reduction in manufacturing costs can be promoted.

### <<Water (F)>>

In addition, the silicone rubber-based curable composition according to the embodiment may include water (F) in addition to the components (A) to (E) .

The water (F) is a component that functions as a dispersion medium for dispersing the respective components in the silicone rubber-based curable composition and contributes to the reaction between the silica particles (C) and the silane coupling agent (D). Therefore, in the silicone rubber, the silica particles (C) and the silane coupling agent (D) can be more reliably linked to each other, and uniform properties can be exhibited as a whole.

### (Other Components)

Further, the silicone rubber-based curable composition according to the embodiment may further include other components in addition to the components (A) to (F). Examples of the other components include an inorganic filler other than the silica particles (C) such as diatomaceous earth, iron oxide, zinc oxide, titanium oxide, barium oxide, magnesium oxide, cerium oxide, calcium carbonate, magnesium carbonate, zinc carbonate, glass wool, or mica, and an additive such as a reaction inhibitor, a dispersant, a pigment, a dye, an antistatic agent, an antioxidant, a flame retardant, or a thermal conductivity enhancing agent.

The conductive solution according to the embodiment include the conductive filler and a solvent in addition to the silicone rubber-based curable composition not including the conductive filler.

As the solvent, various well-known solvents can be used. For example, a high boiling point solvent can be included. As the solvent, one kind may be used alone, or two or more kinds may be used in combination.

Examples of the solvent include: an aliphatic hydrocarbon such as pentane, hexane, cyclohexane, heptane, methylcyclohexane, ethylcyclohexane, octane, decane, dodecane, or tetradecane; an aromatic hydrocarbon such as benzene, toluene, ethylbenzene, xylene, trifluoromethylbenzene, or benzotrifluoride; an ether such as diethyl ether, diisopropyl ether, dibutyl ether, cyclopentyl methyl ether, cyclopentyl ethyl ether, ethylene glycol dimethyl ether, ethylene glycol diethyl ether, diethylene glycol dimethyl ether, 1,4-dioxane, 1,3-dioxane, or tetrahydrofuran; a haloalkane such as dichloromethane, chloroform, 1,1-dichloroethane, 1,2-dichloroethane, 1,1,1-trichloroethane, or 1,1,2-trichloroethane; a carboxylic acid amide such as N,N-dimethylformamide or N,N-dimethylacetamide; and a sulfoxide such as dimethyl sulfoxide or diethyl sulfoxide. As the solvent, one kind may be used alone, or two or more kinds may be used in combination.

By adjusting the solid content in the solution, the conductive solution can be made to have an appropriate viscosity for various coating methods such as spray coating or dip coating.

In addition, in a case where the conductive solution includes the conductive filler and the silica particles (C), the lower limit value of the content of the silica particles (C) in the conductive resin layer 30 is, for example, 1 mass% or higher, preferably 3 mass% or higher, and more preferably 5 mass% or higher with respect to 100 mass% of the total amount of the silica particles (C) and the conductive filler. As a result, the mechanical strength of the conductive resin layer 30 can be improved. On the other hand, the upper limit value of the content of the silica particles (C) in the conductive resin layer 30 is, for example, 20 mass% or lower, preferably 15 mass% or lower, and more preferably 10 mass% or lower with respect to 100 mass% of the total amount of the silica particles (C) and the conductive filler. As a result, a balance between the conductivity and the mechanical strength or flexibility in the conductive resin layer 30 can be realized.

An example of a method of manufacturing the biomedical electrode 100 according to the embodiment may include the following steps.

First, the silicone rubber-based curable composition is molded using a mold by hot press molding to obtain a molded body including the plate-shaped support portion 10 and the pillar portion 20 (molding step).

Next, the distal end portion 26 of the pillar portion 20 is cut in a desired shape to form the inclined surface 28 (inclined surface forming step).

Next, the conductive solution is applied to a surface of the obtained molded body by spray coating and is heated and dried. As a result, the conductive resin layer 30 is formed on the surfaces of the plate-shaped support portion 10 and the pillar portion 20 (conductive resin layer forming step).

Optionally, the distal end portion of the pillar portion 20 may be dipped in the conductive solution and may be heated and dried (distal end coating step).

Next, post-curing (annealing step) is performed at predetermined temperature under temperature conditions.

AS a result, the biomedical electrode 100 can be manufactured.

The inclined surface 28 may be formed on the distal end portion 26 of the pillar portion 20 by molding instead of the cutting method. That is, the molding step and the inclined surface forming step may be performed separately or simultaneously.

The biomedical electrode 100 according to the embodiment can detect a bioelectric signal generated from organic activities of a brain, a heart, a muscle, a nerve, or the like. The biomedical electrode 100 has flexibility and excellent wearability on a scalp, and thus can be suitably used as an electroencephalographic electrode.

The application of an electroencephalographic electrode including the biomedical electrode 100 to a BMI (Brain Machine Interface) is expected.

In addition, the biomedical electrode 100 can be used as a dry sensor that is simple and can be repeatedly used instead of a wet sensor that requires application of gel to a measurement portion. In addition, the biomedical electrode 100 can have flexibility that can reduce pain or discomfort to a subject (user) as compared to a dry sensor of a metal pin type with a spring. In addition, the biomedical electrode 100 can be mounted on a wearable device due to a reduction in size.

A biomedical sensor according to the embodiment will be described.

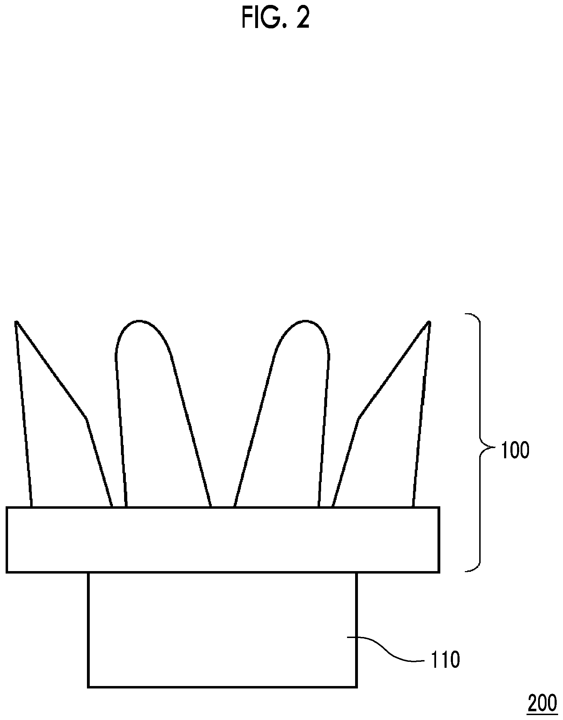

Fig. 2 is a schematic diagram showing the summary of a biomedical sensor 200.

The biomedical sensor 200 according to the embodiment includes the biomedical electrode 100 and may further include an external connection portion 110 connected to the biomedical electrode 100.

The external connection portion 110 may be detachably attached to the plate-shaped support portion 10 of the biomedical electrode 100 or may be fixed to the plate-shaped support portion 10.

From the viewpoint of durability, the external connection portion 110 includes at least an external electrode portion that is more rigid than the silicone rubber and has conductivity. The external electrode portion is formed of, for example, a metal. The external electrode portion can transmit a bioelectric signal detected by the biomedical electrode 100 to an external electronic component. The shape of the external electrode portion is not particularly limited and is configured to be a shape to which a connector or a wiring connectable to an electronic component can be attached. For example, the external connection portion 110 is configured with a metal snap button and has a structure that is electrically connected to an external wiring or an electrode of a substrate through a contact pin.

The biomedical sensor 200 may further include an electronic component that can be electrically connected through the external connection portion 110. As the electronic component, well-known components can be used depending on various uses. Examples of the electronic component include an amplifier, an AD converter, an impedance, a CPU, a memory, a communication circuit, and a battery. Among the examples, one or more components may be modularized on a circuit board. As a result, the biomedical sensor 200 can be used as a wearable device.

In addition, the biomedical sensor 200 may be used in combination with another sensor such as an acceleration sensor or a temperature sensor as the electronic component.

The biomedical sensor 200 includes one or more biomedical electrodes 100. The biomedical sensor 200 may be provided in an attachment jig to a living body such as a headgear or an arm band.

A biomedical signal measurement system according to the embodiment will be described.

The biomedical signal measurement system according to the embodiment includes the biomedical sensor 200. The biomedical signal measurement system may be a system (measurement device) that displays, analyzes, or stores data received from the biomedical sensor 200.

Hereinabove, the embodiment of the present invention has been described. However, the embodiment is an example of the present invention, and various configurations other than the above-described configuration can also be adopted.

Hereinafter, an example of a reference embodiment will be added.
1. A biomedical electrode including:
   a plate-shaped support portion that is formed of a silicone rubber;
   a plurality of pillar portions that protrude from a first surface of the plate-shaped support portion and are formed of a silicone rubber; and
   a conductive resin layer that is formed to cover a distal end of the pillar portion and includes a conductive filler and a silicone rubber,
   in which the plurality of elastic pillar portions are arranged to surround a center portion of the first surface of the plate-shaped support portion, and
   in a cross-sectional view passing through an outside from an inside of the pillar portion with respect to the center portion, the distal end portion of the pillar portion has an inclined surface that is configured such that a height of the pillar portion on the outside is higher than that on the inside.
2. The biomedical electrode according to 1.,
   in which the plurality of pillar portions are arranged on a substantially circumference or a substantially elliptical circumference of the first surface.
3. The biomedical electrode according to 1. or 2.,
   in which a shape of the inclined surface in a top view is a substantially elliptical shape.
4. The biomedical electrode according to any one of 1. to 3.,
   in which the pillar portion is configured to have a substantially truncated conical shape of which a diameter decreases toward the distal end.
5. The biomedical electrode according to any one of 1. to 4.,
   in which roundness is formed at a corner portion where the inclined surface and a side surface of the pillar portion intersect each other.
6. The biomedical electrode according to any one of 1. to 5.,
   in which a content of the conductive filler is 30 mass% or higher and 90 mass% or lower with respect to 100 mass% of the silicone rubber.
7. The biomedical electrode according to any one of 1. to 6.,
   in which the conductive resin layer is configured to cover a second surface of the plate-shaped support portion opposite to the first surface, and
   a thickness of the conductive resin layer at the distal end of the pillar portion is more than a thickness of the conductive resin layer on the second surface of the plate-shaped support portion.
8. The biomedical electrode according to any one of 1. to 7.,
   in which a thickness of the conductive resin layer is 5 µm or more and 200 µm or less.
9. The biomedical electrode according to any one of 1. to 8.,
   in which the conductive filler includes one or more selected from the group consisting of metal particles, metal fiber, metal-coated fiber, carbon black, acetylene black, graphite, carbon fiber, carbon nanotube, a conductive polymer, conductive polymer-coated fiber, and metal nanowire.
10. The biomedical electrode according to any one of 1. to 9.,
   in which the biomedical electrode is used as an electroencephalographic electrode.
11. A biomedical sensor including the biomedical electrode according to any one of 1. to 10.
12. A biomedical signal measurement system including the biomedical sensor according to 11.

### [Examples]

Hereinafter, examples of the present invention will be described in detail. However, the present invention is not limited to the description of the examples.

Raw material components shown in Table 1 are as described below.

### (Vinyl Group-Containing Organopolysiloxane (A))

(A1-1): first vinyl group-containing linear organopolysiloxane: vinyl group content = 0.13 mol%, Mn = 227,734, Mw = 573,903, IV value (dl/g) = 0.89), a vinyl group-containing dimethylpolysiloxane (the structure represented by Formula (1-1)) synthesized according to the following synthesis scheme 1
(A1-2): second vinyl group-containing linear organopolysiloxane: vinyl group content = 0.92 mol%, a vinyl group-containing dimethylpolysiloxane (the structure represented by Formula (1-1) where R¹ and R² represent a vinyl group) synthesized according to the following synthesis scheme 2

### (Organohydrogen Polysiloxane (B))

(B): organohydrogen polysiloxane: manufactured by Momentive Inc. "TC-25D"

### (Silica Particles (C))

(C): Silica fine particles (particle size: 7nm, specific surface area: 300 m²/g) , manufactured by Nippon Aerosil Co., Ltd., "AEROSIL 300"

### (Silane Coupling Agent (D))

(D-1): Hexamethyldisilazane (HMDZ), manufactured by Gelest Inc. "HEXAMETHYLDISILAZANE" (SIH6110.1)"
(D-2): divinyltetramethyldisilazane, manufactured by Gelest Inc., "1,3-DIVINYLTETRAMETHYLDISILAZANE (SID4612.0)"

### (Platinum or Platinum Compound (E))

(E) : platinum or a platinum compound: manufactured by Momentive Inc., "TC-25A"

### (Water (F))

(F): pure water

### (Metal Powder (G))

(G1) : silver powder, manufactured by Tokuriki Honten Co., Ltd, trade name "TC-101", median size d₅₀: 8.0 µm, aspect ratio: 16.4, average long diameter: 4.6 µm

### (Synthesis of Vinyl Group-Containing Organopolysiloxane (A))

### [Synthesis scheme 1: Synthesis of First Vinyl Group-Containing Linear Organopolysiloxane (A1-1) ]

The first vinyl group-containing linear organopolysiloxane (A1-1) was synthesized according to the following Formula (5).

That is, 74.7 g (252 mmol) of octamethylcyclotetrasiloxane and 0.1 g of potassium siliconate were added to 300 mL separable flask including a cooling tube and a mixing impeller where the atmosphere was replaced with Ar gas. The solution was heated and stirred at 120°C for 30 minutes. At this time, an increase in viscosity was observed.

Next, the solution was heated up to 155°C and was continuously stirred for 3 hours. After 3 hours, 0.1 g (0.6 mmol) of 1,3-divinyltetramethyldisiloxane was added and was further stirred at 155°c for 4 hours.

Further, after 4 hours, the solution was diluted with 250 mL of toluene, and was cleaned with water 3 times. After cleaning, the organic layer was cleaned with 1.5 L of methanol and was purified by reprecipitation to separate an oligomer and a polymer. The obtained polymer was dried at 60 °C under reduced pressure overnight. As a result, the first vinyl group-containing linear organopolysiloxane (A1-1) was obtained (Mw= 573, 903, Mn = 227, 734) . In addition, the vinyl group content calculated by H-NMR spectrum measurement was 0.13 mol%.

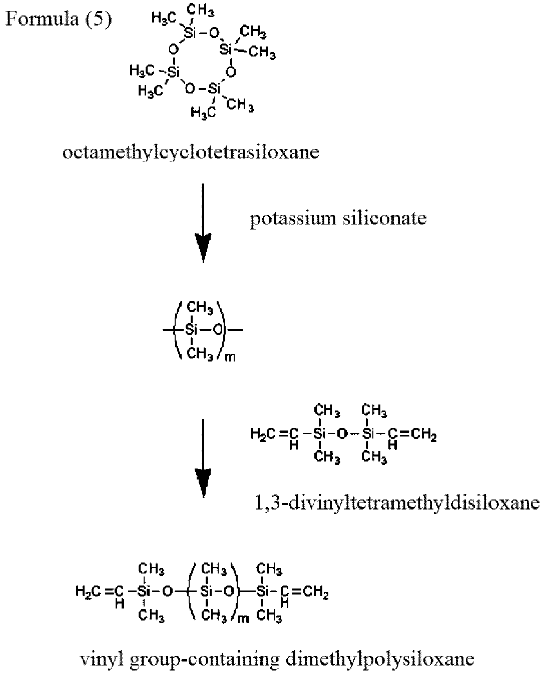

### [Synthesis scheme 2: Synthesis of Second Vinyl Group-Containing Linear Organopolysiloxane (A1-2) ]

The second vinyl group-containing linear organopolysiloxane (A1-2) was synthesized as shown in the following Formula (6) using the same method as the synthesis step of (A1-1), except that 74.7 g (252 mmol) of octamethylcyclotetrasiloxane and 0.86 g (2.5 mmol) of 2,4,6,8-tetraethenyl-2,4,6,8-tetravinylcyclotetrasiloxane were used in the synthesis step of (A1-1). In addition, the vinyl group content calculated by H-NMR spectrum measurement was 0.92 mol%.

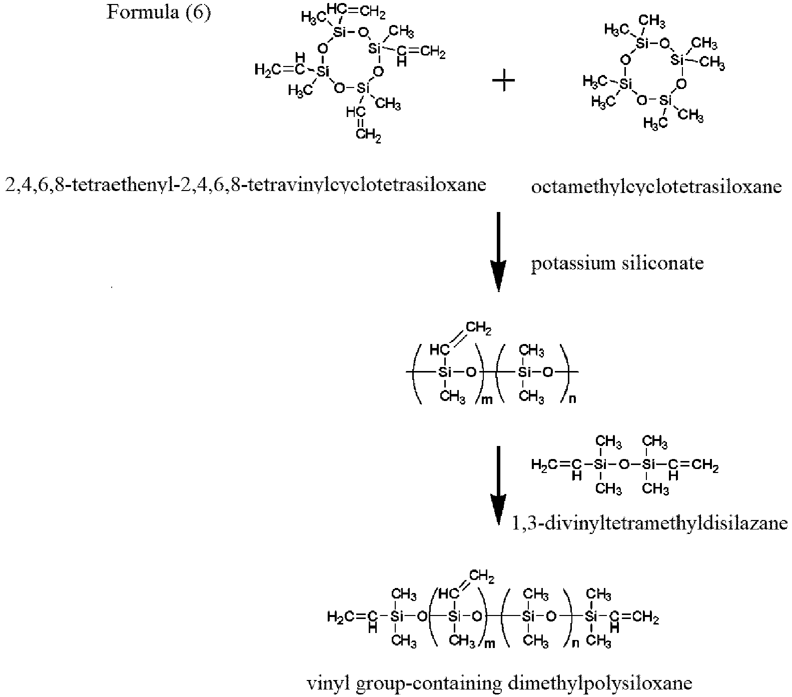

### <Preparation of Silicone Rubber-Based Curable Composition>

The silicone rubber-based curable composition was prepared as follows.

First, a mixture including 90% of the vinyl group-containing organopolysiloxane (A), the silane coupling agent (D) , and the water (F) at a ratio shown in Table 1 below was kneaded in advance. Next, the silica particles (C) were further added to the mixture and kneaded. As a result, a kneaded material (silicone rubber compound) was obtained.

Here, the kneading after the addition of the silica particles (C) was performed through a first step of kneading the components under conditions of 60°C to 90°C in a nitrogen atmosphere for the coupling reaction and a second step of kneading the components under conditions of 160°C to 180°C for 2 hours in a reduced pressure atmosphere for removing the by-product (ammonia) . Next, the kneaded material was cooled, the remaining 10% of the vinyl group-containing organopolysiloxane (A) was dividedly added twice, and the components were kneaded for 20 minutes.

Next, the organohydrogen polysiloxane (B) and the platinum or platinum compound (E) were added to 100 parts by weight of the obtained kneaded material (silicone rubber compound) at a ratio shown in Table 1 below, and the components were kneaded with a roll. As a result, the silicone rubber-based curable composition A (elastomer composition) was obtained.

**[Table 1]**

| | | | | Unit | A |
|---|---|---|---|---|---|
| Silicone Rubber-Based Curable Composition | Silicone Rubber Compound (Kneaded Material) | Vinyl Group-Containing Organopolysiloxane (A) | (A1-1) | Part(s) by Weight | 80 |
| | | | (A1-2) | | 20 |
| | | Silica Particles (C) | | | 25 |
| | | Silane Coupling Agent (D) | (D-1) | | 10.0 |
| | | | (D-2) | | 0.5 |
| | | Water (F) | | | 5.25 |
| | Catalyst | Platinum or Platinum Compound (E) | | Part(s) by Weight (with respect to 100 Parts by Weight of Kneaded Material) | 0.5 |
| | Crosslinking Agent | Organohydrogen Polysiloxane (B) | | | 2.64 |

### <Preparation of Conductive Solution for Spray Coating>

13.7 parts by weight of the obtained silicone rubber-based curable composition A was dipped in 31.8 parts by weight of decane (solvent), the solution was stirred using a rotating and revolving mixer, 54.5 parts by weight of metal powder (G1) was added, and the components were kneaded with three rolls. As a result, a resin varnish was obtained. Next, decane was added to the resin varnish such that the amount of decane was 2.5 times that of the resin varnish, and was stirred using a rotating and revolving mixer to dilute the resin varnish. As a result, a conductive solution for spray coating was obtained.

### <Preparation of Conductive Solution for Dip Coating>

13.7 parts by weight of the obtained silicone rubber-based curable composition A was dipped in 31.8 parts by weight of decane (solvent), the solution was stirred using a rotating and revolving mixer, 54.5 parts by weight of metal powder (G1) was added, and the components were kneaded with three rolls. As a result, a conductive paste (conductive solution for dip coating) was obtained.

### <Preparation of Biomedical Electrode>

### (Example 1)

The silicone rubber-based curable composition A was heated and cured at 180°C and 10 MPa for 10 minutes using a mold including a molding space for the plate-shaped support portion and six pillar portions. As a result, a molded body where the plate-shaped support portion and the six pillar portions were integrated was obtained (molding step).

Next, the distal end portions of all the pillar portions were obliquely cut to form an inclined surface in the distal end portion (inclined surface forming step: cutting step).

<Conductive Solution for Spray Coating> described above was applied to the entire surface of the obtained molded body by spray coating and was heated and dried at 120°C for 30 minutes. As a result, a conductive resin layer was formed on the surface of the molded body (conductive resin layer forming step).

Next, only the distal end portion of the pillar portion was dipped in <Conductive Solution for Dip Coating> and was heated and dried at 120°C for 30 minutes (distal end coating step).

Next, post-curing was performed at 180°C for 2 hours (annealing step).

As a result, a biomedical electrode A shown in Fig. 1A was obtained. The distal end angle (the inclination angle θ of the inclined surface) shown in Fig. 1B was 33 degrees.

### (Example 2)

A biomedical electrode B was obtained using the same method as that of Example 1, except that the angle at which the distal end portion was obliquely cut was changed in the cutting step of Example 1. The distal end angle (the inclination angle θ of the inclined surface) shown in Fig. 1B was 43 degrees.

### (Comparative Example 1)

A biomedical electrode C was obtained using the same method as that of Example 1, except that the cutting step of Example 1 was not performed. All the pillar portions did not have inclined surfaces in the distal end portions, the region from the base end to the distal end was formed in a pillar shape, and the distal end was flat.

The obtained biomedical electrodes A to C were evaluated for the following evaluation items. The evaluation results are shown in Table 2.

**[Table 2]**

| | Unit | Example 1 | Example 2 | Comparative Example 1 |
|---|---|---|---|---|
| Biomedical Electrode | | A | B | C |
| Inclined Surface of Distal End Portion | | Provided | Provided | Not Provided |
| Distal End Angle | Degree | 33 | 43 | - |
| Brain Wave Acquisition Success Ratio | % | 100 | 100 | 20 |
| Measurement Stability | | A | A | D |

In Table 1, regarding the distal end angle of Comparative Example 1, the inclined surface was not provided, but it can be assumed that θ in Fig. 1B was 90 degrees.

### (Measurement Stability)

### <Preparation of Electroencephalographic System)

As shown in Fig. 2, the external connection portion 110 (a metal snap button having a structure where an end portion of a cable is mountable) was mounted on the second surface 14 of the biomedical electrode 100 obtained in <Preparation of Biomedical Electrode> described above. A 1ch preamplifier cable (manufactured by Unique Medical Co., Ltd., trade name: TF217-026), a biomedical amplifier (manufactured by Unique Medical Co., Ltd., trade name: a potable biomedical amplifier, EBA-100), and an oscilloscope were electrically connected to the external connection portion 110 in this order to prepare an electroencephalographic system.

Next, a headgear (a headgear that has a node arrangement according to the ten-twenty electrode system of the International Federation that was molded using a 3D printer) for electroencephalography was worn in the head of a subject.

Next, the top of the head of the subject was brought into contact with the distal end portion of the biomedical electrode 100 to determine whether or not a brain wave was able to be obtained based on a waveform displayed on a monitor of the oscilloscope (electroencephalography procedure).

This electroencephalography procedure was repeatedly performed on the same subject five times (measurement times), and the number of times (the number of times of brain wave acquisition success) a brain wave was able to be measured was recorded. A ratio (%) of the number of times of brain wave acquisition success to the measurement times was set as "brain wave acquisition success ratio". The results are shown in Table 2.

When a brain wave was able to be measured, the baseline was constant, and the waveform was stable.

The measurement stability of the biomedical electrode was evaluated based on the following evaluation criteria. The results are shown in Table 2.

A case where the brain wave acquisition success ratio was 80% or higher and 100% or lower was evaluated as "A", a case where the brain wave acquisition success ratio was 60% or higher and lower than 80% was evaluated as "B", a case where the brain wave acquisition success ratio was 40% or higher and lower than 60% was evaluated as "C", and a case where the brain wave acquisition success ratio was 0% or higher and lower than 40% was evaluated as "D".

### <Deformation Stability>

The distal ends of the plurality of pillar portions of the biomedical electrode obtained in <Preparation of Biomedical Electrode> described above were pressed against a spherical surface of a transparent semi-spherical dome (a glass dome having a surface on which a silicon sheet was formed) to observe deformation directions of the plurality of pillar portions. All the distal ends of the six pillar portions were simultaneously brought into contact with and were pressed against the spherical surface such that the same load was applied thereto.

In the biomedical electrodes according to Examples 1 and 2, all the pillar portions were deformed to spread from the center portion toward the outside direction of the circle.

On the other hand, in the biomedical electrode according to Comparative Example 1, some pillar portions were deformed from the center portion toward the outside direction of the circle, but other pillar portions were deformed from the outside toward the center portion.

It was found that, in the biomedical electrodes according to Examples 1 and 2, the deformation stability was higher than that of Comparative Example 1, and the measurement stability was also excellent.

The present application claims priority based on Japanese patent application NO. 2018-202286 filed on October 26, 2018, the entire contents of which are incorporated herein by reference.

## Claims

1. A biomedical electrode comprising:
a plate-shaped support portion;
a plurality of elastic pillar portions that are provided on a first surface of the plate-shaped support portion; and
a conductive resin layer that is formed to cover distal ends of the elastic pillar portions,
wherein the plurality of elastic pillar portions are arranged to surround a center portion of the first surface of the plate-shaped support portion,
each of distal end portions of the plurality of elastic pillar portions has an inclined surface that is inclined with respect to the first surface, and
when a point of the inclined surface closest to the first surface is represented by P1, a point of the inclined surface farthest from the first surface is represented by P2, and a half line extending from P2 to P1 is represented by L,
the point P1 is present at a position closer to the center portion than the point P2 in the plurality of elastic pillar portions, and
the half line L is configured to pass through the center portion or a vicinity of the center portion in the plurality of elastic pillar portions.

2. The biomedical electrode according to claim 1,
wherein each of the half lines L is configured to intersect the center portion in the plurality of elastic pillar portions.

3. The biomedical electrode according to claim 1 or 2,
wherein the distal end portion of the elastic pillar portion does not have a flat surface parallel to the first surface.

4. The biomedical electrode according to any one of claims 1 to 3,
wherein the inclined surface of the elastic pillar portion and a side surface of the elastic pillar portion are configured to intersect each other through the point P1.

5. The biomedical electrode according to any one of claims 1 to 4,
wherein a shape of the inclined surface in a top view is substantially elliptical when seen from the distal end side toward a base end side of the elastic pillar portion.

6. The biomedical electrode according to any one of claims 1 to 5,
wherein a corner portion is formed at a position where the inclined surface of the elastic pillar portion and the side surface of the elastic pillar portion intersect each other, and
the corner portion is rounded.

7. The biomedical electrode according to any one of claims 1 to 6,
wherein the elastic pillar portion is configured to have a substantially truncated conical shape of which a diameter decreases toward the distal end.

8. The biomedical electrode according to any one of claims 1 to 7, wherein the plurality of elastic pillar portions are arranged on the first surface in a substantially circular shape or a substantially elliptical shape.

9. The biomedical electrode according to any one of claims 1 to 8,
wherein the elastic pillar portion is formed of an insulating silicone rubber including a silicone rubber.

10. The biomedical electrode according to any one of claims 1 to 9,
wherein the conductive resin layer is formed of a conductive silicone rubber including a conductive filler and a silicone rubber.

11. The biomedical electrode according to claim 10,
wherein a content of the conductive filler is 30 mass% or higher and 90 mass% or lower with respect to 100 mass% of the silicone rubber.

12. The biomedical electrode according to claim 10 or 11,
wherein the conductive filler includes one or more selected from the group consisting of metal particles, silver or silver chloride particles, metal fiber, metal-coated fiber, carbon black, acetylene black, graphite, carbon fiber, carbon nanotube, a conductive polymer, conductive polymer-coated fiber, and metal nanowire.

13. The biomedical electrode according to any one of claims 1 to 12,
wherein the conductive resin layer is configured to cover a second surface of the plate-shaped support portion opposite to the first surface, and
a thickness of the conductive resin layer at the distal end of the elastic pillar portion is more than a thickness of the conductive resin layer on the second surface of the plate-shaped support portion.

14. The biomedical electrode according to any one of claims 1 to 13,
wherein a thickness of the conductive resin layer positioned at the distal end of the elastic pillar portion is 5 µm or more and 200 µm or less.

15. The biomedical electrode according to any one of claims 1 to 14,
wherein the biomedical electrode is used as an electroencephalographic electrode.

16. A biomedical sensor comprising the biomedical electrode according to any one of claims 1 to 15.

17. A biomedical signal measurement system comprising the biomedical sensor according to claim 16.
